Europäisches Patentamt

(19) European Patent Office

Office européen des brevets

(11) Numéro de publication: **0 141 001
B1**

(12) **FASCICULE DE BREVET EUROPEEN**

(45) Date de publication du fascicule du brevet:
**28.12.88**

(51) Int. Cl.⁴: **G 01 N 33/92,** G 01 N 33/53,
G 01 N 33/561

(21) Numéro de dépôt: **83401974.7**

(22) Date de dépôt: **11.10.83**

(54) **Dosage immunologique dans le sérum de l'apolipoprotéine B des lipoprotéines de basse densité.**

(43) Date de publication de la demande:
**15.05.85 Bulletin 85/20**

(45) Mention de la délivrance du brevet:
**28.12.88 Bulletin 88/52**

(84) Etats contractants désignés:
**AT BE CH DE GB IT LI LU NL SE**

(56) Documents cités:
**WO-A-79/00044
WO-A-81/02790
GB-A- 1 601 436
GB-A- 2 041 003
US-A- 4 147 606**

**CLINICAL CHEMISTRY, vol. 28, no. 1, janvier 1982, pages 59-62, Easton, Penn., US; J.-C. FRUCHART et al.: "Simultaneous measurement of plasma apolipoproteins A-i and B by electroimmunoassay"
CLINICAL CHEMISTRY, vol. 27, no. 6, juin 1981, pages 892-895, Easton, Penn., US; M.F. REARDON et al.: "Improved method for quantitation of B apoprotein in plasma lipoproteins by electroimmunoassay"**

(73) Titulaire: **SPIRAL Société à responsabilité limitée dite :,
3, rue des Mardors Z.A. Couternon BP 1408,
F-21051 Dijon Cédex (FR)**

(72) Inventeur: **Gambert, Philippe, 6, rue Claude Boucru,
F-21000 Dijon (FR)**
Inventeur: **Louvrier, Emmanuel, 37, rue J.B. Bavain,
F-21000 Dijon (FR)**

(74) Mandataire: **Gillard, Marie-Louise et al, Cabinet Beau de
Loménie 55, Rue d'Amsterdam, F-75008 Paris (FR)**

## Description

L'invention a pour objet un procédé permettant le dosage immunologique d'une seule espèce protéique au sein d'une famille de protéines ou de complexes protéiques présentant un déterminant antigénique commun, mais de tailles différentes.

Le procédé selon l'invention permet d'effectuer successivement, en une seule opération utilisant un nouveau système électrophorétique, une séparation des protéines selon leur taille dans un milieu de réticulation contrôlée, et un dosage par électro-immunodiffusion selon la méthode de LAURELL, C.B. Anal. Biochem. 15 45–52 (1966). Le milieu de réticulation contrôlée dans lequel s'effectue la migration électrophorétique préalable permet le blocage des protéines de taille supérieure à celle de la protéine à doser, de façon que ces protéines ne puissent interférer dans le dosage par électro-immunodiffusion ultérieur.

L'invention a en particulier pour objet un procédé de dosage dans le sérum, de l'apolipoprotéine B (désignée ci-après apo-B) se trouvant dans des lipoprotéines de faible densité (ci-après LDL).

L'apolipoprotéine B (apo B) est une protéine constitutive de différents complexes lipoprotéines sériques: les lipoprotéines de très faible densité (VLDL), les lipoprotéines de densité intermédiaire (IDL) et les lipoprotéines de faible densité (LDL), complexes de tailles, de compositions chimiques, de structures et de rôles physiopathologiques très différents.

GOLDSTEIN J.L. et BROWN M.S., Ann. Rev. Biochem. 46 897–930 (1977) ont montré que l'apo B des LDL règle le métabolisme cellulaire des lipoprotéines sériques et ont suggéré qu'elle jouerait ainsi un rôle clef dans la survenue du processus athéroscléreux. Des études cliniques récentes [SNIDERMAN A. et al., Proc. Nat. Acad. Sci. U.S.A. 77 604–608 (1980)] ont confirmé ces données expérimentales en montrant le caractère discriminant de la concentration sérique de l'apo B des LDL dans le diagnostic et le pronostic des coronopathies.

Il est à noter de plus que les LDL forment une famille moléculaire hétérogène dans laquelle il est possible d'individualiser plusieurs fractions [KRAUSS R.M. et BURKE D.J.J. Lipid. Res. 23 97–104 (1982)] et notamment la lipoprotéine Lpa de constitution particulière et dont la responsabilité dans le développement précoce de l'athérosclérose a été suspectée [KOSTNER G.M. et al. Atherosclerosis 38 51–61 (1981)].

Les méthodes de dosage de l'apo B actuellement proposées dosent toutes l'apo B sérique totale sans différencier l'apo B selon son origine et donc sa signification physiopathologique. Un dosage de l'apo B des différentes fractions lipoprotéiques a bien été proposé par SNIDERMAN et al. (cités ci-dessus), mais il repose sur la séparation des lipoprotéines par ultracentrifugation préparative, méthode longue, coûteuse et non réellement quantitative.

Le procédé selon l'invention permet le dosage de l'apo B des LDL totales sans interférence des autres lipoprotéines. Une variante du procédé permet d'éliminer du dosage l'apo B liée à la fraction Lpa.

Sous l'action d'un champ électrique sont effectués successivement, en une seule opération:
- une séparation des LDL des autres lipoprotéines contenant de l'apo B;
- un dosage de l'apo B des LDL par électro-immunodiffusion selon la méthode de LAURELL C.B. (cité ci-dessus).

La première étape, séparative, est réalisée par migration électrophorétique des échantillons dans un gel de polyacrylamide ou d'un polymère équivalent, dont la réticulation est telle qu'il y ait passage des LDL et rétention des autres lipoprotéines à apo B, en particulier les VLDL qui ont toutes une taille supérieure à celle des LDL. On contrôle donc la réticulation du gel au cours de sa préparation de façon que le gel obtenu bloque toute protéine étalon d'un diamètre supérieur à 30 nm.

La deuxième étape de dosage est réalisée par migration électrophorétique des LDL dans un gel d'agarose contenant des anticorps anti-apo B.

L'étape séparative rend compte de la spécificité du dosage. Elle améliore également sa précision et sa linéarité. En effet, ce qui limite les performances des techniques d'électro-immunodiffusion est l'existence, avant toute migration électrophorétique, d'une diffusion passive des échantillons dans l'agarose, diffusion variable d'un dépôt à l'autre, qui retentit sur la hauteur des arcs de précipitation des immuns complexes et qui de ce fait entraîne des défauts de reproductibilité et de linéarité. Il a parfois été proposé de pallier cet inconvénient en pratiquant les dépôts alors que la plaque est sous tension [AYRAULT-JARRIER M. Ann. Biol. Clin. 40 187–194 (1982)], ce qui n'est ni parfaitement efficace, ni compatible avec les règles de sécurité. Dans le procédé selon l'invention, les échantillons sont déposés à l'intérieur de puits pratiqués dans le gel d'acrylamide ce qui rend toute diffusion impossible et supprime les défauts signalés.

Selon une variante du procédé selon l'invention, on exclut du dosage l'apo B liée à la lipoprotéine Lpa en introduisant un gel supplémentaire entre le gel de séparation et le gel d'électro-immunodiffusion. Il s'agit d'un gel d'acrylamide contenant des anticorps spécifiques de la lipoprotéine Lpa. Lors de la migration des LDL dans ce gel, les lipoprotéines Lpa forment, avec les anticorps correspondants, des immuns complexes de taille telle que leur migration devient impossible. Seules les autres LDL continuent leur migration en direction du gel d'électro-immunodiffusion.

Le procédé selon l'invention est mis en œuvre par l'emploi d'un nouvel élément analytique composé d'une plaque (ou d'un film) constituée pour une partie, d'un gel d'acrylamide-agarose et pour une autre partie, d'un gel d'agarose. La plaque (ou film) utilisée comme support horizontal de la migration électrophorétique, comprend:

a)–Du côté cathodique, une première partie constituée d'un gel de polyacrylamide contenant une faible proportion d'agarose ayant pour but d'assurer la tenue mécanique du gel, et qui peut varier de 0,05 à 5% en poids du gel.

Pour la préparation de ce gel, on utilise, au cours de la polymérisation, des agents de réticulation classiques, tels que le méthylène-bis-acrylamide ou le di-allyl-tartar-diamide, de façon à obtenir un gel qui ne laisse pas passer les protéines étalon d'un diamètre supérieur à 30 nm.

Si, par exemple, on part d'un mélange à 30 g/litre d'acrylamide et 7 g/litre d'agarose, on ajoute l'agent de réticulation à raison de 10% en poids par rapport à l'acrylamide. Des puits destinés au dépôt des échantillons sont ménagés dans ce premier gel à une distance de quelques millimètres, 2 mm par exemple, de l'interface des deux gels.

b)–Du côté anodique, une seconde partie, constituée d'un gel d'agarose (par exemple 10 g/l) contenant l'anticorps anti-apo B.

En vue de suivre la migration et la précipitation des lipoprotéines, les échantillons sont mélangés volume à volume à une solution de Noir Soudan (5 g/l dans l'éthylèneglycol), colorant des lipides.

Un microlitre de chaque mélange est déposé dans le puits réservé à cet effet.

Une différence de potentiel de 100 V est maintenue pendant 3 heures entre les extrémités de la plaque.

On mesure ensuite les hauteurs ou les surfaces des arcs de précipitation des immuns complexes. Les concentrations en apo B des échantillons sont calculées par comparaison avec les hauteurs obtenues avec des préparations de concentrations connues en apo B des LDL.

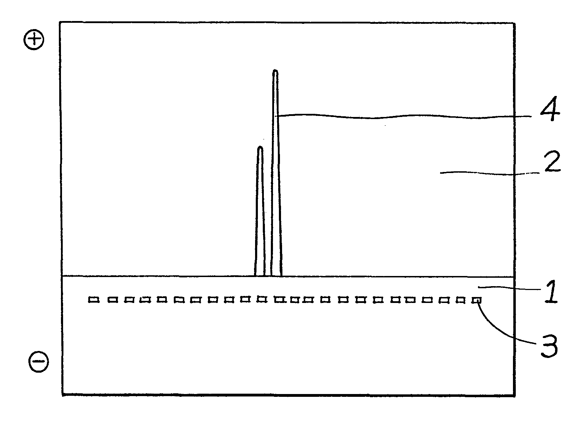

La figure unique ci-après représente un élément analytique selon l'invention, comportant un gel 1 assurant le blocage des VLDL et un gel 2 contenant l'antisérum anti-apo B; les échantillons sont déposés dans les puits 3 et on voit en 4 les arcs de précipitation des immuns complexes.

Selon une variante du procédé selon l'invention, on exclut du dosage la lipoprotéine Lpa:

Entre les deux gels précédemment décrits est disposé un troisième gel d'acrylamide-agarose (par exemple, acrylamide: 30 g/l, agarose: 7 g/l) contenant des anticorps anti-Lpa.

Le mode opératoire est par ailleurs identique à celui décrit ci-dessus.

Le procédé selon l'invention permet de doser l'apo B des LDL avec une bonne reproductibilité: c.v. inférieur à 1% sur une même plaque (n = 20). La linéarité dépend du sérum anti-apo B utilisé dans le gel d'électro-immunodiffusion. Avec un anti-sérum d'origine commerciale, la linéarité s'étend de 0,3 à 1,8 g/l d'apo B.

Le procédé de dosage selon l'invention présente également une bonne spécificité: des surcharges croissantes en VLDL du sérum à doser n'ont pas entraîné de modifications significatives des hauteurs de pics.

Le procédé décrit pour le dosage spécifique de l'apo B d'une seule fraction lipoprotéique peut être appliqué au dosage d'une seule espèce protéique au sein d'ensembles protéiques dont les constituants possèdent un déterminant antigénique commun, mais sont de tailles différentes.

**Revendications**

1. Procédé de dosage de l'apolipoprotéine B (apo B) des lipoprotéines de basse densité (LDL) contenues dans le sérum, milieu dans lequel les LDL sont en mélange avec d'autres lipoprotéines contenant de l'apo B, caractérisé en ce que l'on réalise tout d'abord une séparation des LDL des autres lipoprotéines contenant de l'apo B par migration électrophorétique à travers une couche de gel de polyacrylamide ou d'un polymère équivalent dont le degré de réticulation est contrôlé de façon à bloquer la migration de toute lipoprotéine d'un diamètre supérieur à 30 nm, et l'on procède ensuite au dosage de l'apo B des LDL ainsi séparées, dès leur sortie de la couche de séparation, par électroimmunodiffusion dans une couche de gel d'agarose contenant des anticorps anti-apo B, la séparation par électrophorèse et le dosage par électroimmunodiffusion étant effectués à l'aide d'une plaque ou d'un film d'électrophorèse dont la partie cathodique est constituée par ladite couche de gel de polyacrylamide à degré de réticulation contrôlé, et dont la partie anodique adjacente à la partie cathodique est constituée par ladite couche de gel d'agarose contenant des anticorps anti-apo B.

2. Procédé selon la revendication 1, caractérisé en ce que le gel de polyacrylamide est réticulé par des agents tels que le méthylène-bis-acrylamide et le di-allyl-tartar-diamide.

3. Procédé selon l'une des revendications 1 à 2, caractérisé en ce que l'on évite la diffusion passive des échantillons en déposant ceux-ci dans des puits pratiqués dans le gel de polyacrylamide et situés à quelques millimètres de l'interface des deux gels.

4. Procédé selon l'une des revendications 1 à 3, caractérisé en ce que le gel de polyacrylamide, constituant une partie de la plaque d'électrophorèse, contient une proportion d'agarose suffisante pour assurer la tenue mécanique du gel, et en particulier une proportion de 0,05 à 5% en poids du gel.

5. Procédé selon l'une des revendications 1 à 4, caractérisé en ce que, pour exclure du dosage l'apo B liée à la lipoprotéine Lpa, on introduit, dans la plaque d'électrophorèse, entre le gel de séparation et le gel d'électro-immunoprécipitation, un gel supplémentaire constitué par un polyacrylamide de réticulation contrôlée contenant des anticorps spécifiques de la lipoprotéine Lpa.

6. Plaque ou film d'électrophorèse destiné à mettre en œuvre le procédé de dosage selon l'une des revendications 1 à 5, caractérisé en ce qu'il comprend, du côté cathodique, une couche de gel de polyacrylamide ou d'un polymère équivalent dont le degré de réticulation est contrôlé de façon à bloquer la migration de toute lipoprotéine d'un diamètre supérieur à 30 nm, et du côté anodique une couche de gel d'agarose contenant des anticorps anti-apo B.

7. Plaque ou film d'électrophorèse selon la revendication 6, caractérisé en ce qu'il comporte une couche intermédiaire de gel de polyacrylamide de degré de réticulation contrôlé, contenant des anticorps spécifiques de la lipoprotéine Lpa, cette couche intermédiaire étant intercalée entre la couche de séparation et la couche d'électroimmunodiffusion.

**Claims**

1. Method of determining the apolipoprotein B (apo B) of low density lipoproteins (LDL) contained in serum in which the LDLs are mixed with other lipoproteins containing apo B, characterized in that it consists first in separating the LDLs from the other lipoproteins containing the apo B by electrophoretic migration through a layer of polyacrylamide gel or an equivalent polymer gel of which the crosslinking degree is controlled so as to block the migration of any lipoprotein of diameter greater than 30 nm, and then in determining the apo B of the thus separated LDLs, on their emerging from the separation layer, by electroimmunodiffusion in an agarose gel layer containing anti-apo B antibodies, the separation by electrophoresis and the determination by electroimmunodiffusion being carried out by means of an electrophoresis plate or film of which the cathodic portion is constituted by said polyacrylamide gel layer with said controlled degree of reticulation, and of which the anodic part adjacent to the cathodic part is constituted by said agarose gel layer containing anti-apo B antibodies.

2. Method according to claim 1, characterized in that the polyacrylamide gel is crosslinked by agents such as methylene-bis-acrylamide and di-allyl-tartar-diamide.

3. Method according to one of claims 1 and 2, characterized in that the passive diffusion of the samples is avoided by depositing the latter in wells formed in the polyacrylamide gel and situated at some millimeters from the interface of the two gels.

4. Method according to one of claims 1 to 3, characterized in that the polyacrylamide gel, constituting a portion of the electrophoresis plate, contains a sufficient proportion of agarose to ensure the mechanical strength of the gel, in particular a proportion from 0.05 to 5% by weight of the gel.

5. Method according to one of claims 1 to 4, characterized in that, in order to exclude from the assay the apo B coupled to the lipoprotein Lpa, there is introduced into the electrophoresis plate, between the separating gel layer and the electroimmunodiffusion gel layer, an additional polyacrylamide gel layer of said controlled degree of reticulation containing specific antibodies of lipoproteins Lpa.

6. Electrophoresis plate or film for carrying out the assaying method according to one of claims 1 to 5, characterized in that it comprises, on the cathodic side, a layer of polyacrylamide gel or an equivalent polymer gel of which the degree of cross-linking is controlled so as to block the migration of any lipoprotein of diameter greater than 30 nm, and on the anodic side a layer of agarose gel containing anti-apo B antibodies.

7. Electrophoresis plate or film according to claim 6, characterized in that it comprises an intermediate layer of polyacrylamide gel of said controlled degree of reticulation, containing specific antibodies of lipoprotein Lpa, this intermediate layer being inserted between the separation layer and the electro-immunodiffusion layer.

**Patentansprüche**

1. Verfahren zur Bestimmung von Apolipoprotein B (Apo B) von Lipoproteinen niedriger Dichte (LDL), die im Serum enthalten sind, einem Milieu, in welchem die LDL in Mischung mit anderen Apo B enthaltenden Lipoproteinen vorhanden sind, dadurch gekennzeichnet, dass man zuerst eine Abtrennung der LDL von den anderen Apo B enthaltenden Lipoproteinen mittels elektrophoretischer Migration durch eine Gelschicht aus Polyacrylamid oder einem äquivalenten Polymeren, dessen Vernetzungsgrad derart gesteuert ist, dass die Migration jedes Lipoproteins mit einem Durchmesser von mehr als 30 nm blockiert wird, ausführt und man danach die Bestimmung des Apo B der so abgetrennten LDL, sobald diese die Abtrennschicht verlassen haben, durch Elektroimmundiffusion in eine Agarosegelschicht, enthaltend anti-Apo B-Antikörper, vornimmt, wobei die Abtrennung mittels Elektrophorese und die Bestimmung mittels Elektroimmundiffusion mit Hilfe einer Elektrophoreseplatte oder eines -films durchgeführt werden, dessen Kathodenteil durch die Polyacrylamidgelschicht mit gesteuertem Vernetzungsgrad gebildet ist und dessen Anodenteil neben dem Kathodenteil durch die anti-Apo B-Antikörper enthaltende Agaroseschicht gebildet ist.

2. Verfahren nach Anspruch 1, dadurch gekennzeichnet, dass das Polyacrylamidgel durch Mittel, wie Methylen-bis-acrylamid und Di-allyltartardiamid vernetzt wird.

3. Verfahren nach einem der Ansprüche 1 bis 2, dadurch gekennzeichnet, dass man die passive Diffusion der Proben vermeidet, indem man diese in im Polyacrylamidgel geschaffene Gruben, die sich einige Millimeter von der Grenzfläche der beiden Gele befinden, gibt.

4. Verfahren nach einem der Ansprüche 1 bis 3, dadurch gekennzeichnet, dass das Polyacrylamidgel, das einen Teil der Elektrophoreseplatte darstellt, einen Anteil an Agarose enthält, der ausreicht, den mechanischen Halt des Gels zu gewährleisten, insbesondere einen Anteil von 0,05 bis 5 Gew.-% des Gels.

5. Verfahren nach einem der Ansprüche 1 bis 4, dadurch gekennzeichnet, dass man zwecks Ausschlusses der Bestimmung von an Lipoprotein Lpa gebundenem Apo B in die Elektrophoreseplatte zwischen das Abtrenngel und das Elektroimmunpräzipitationsgel ein zusätzliches Gel einbringt, das durch ein Polyacrylamid mit kontrollierter Vernetzung, enthaltend spezifische Antikörper des Lipoproteins Lpa, gebildet ist.

6. Elektrophoreseplatte oder -film zur Durchführung des Bestimmungsverfahrens nach einem der Ansprüche 1 bis 5, dadurch gekennzeichnet, dass er auf der Kathodenseite eine Gelschicht aus Polyacrylamid oder einem äquivalenten Polymeren, dessen Vernetzungsgrad derart gesteuert ist, dass die Migration jedes Lipoproteins mit einem Durchmesser von mehr als 30 nm blockiert wird, und auf der Anodenseite eine Agarosegelschicht, enthaltend anti-Apo B-Antikörper, umfasst.

7. Elektrophoreseplatte oder -film nach Anspruch 6, dadurch gekennzeichnet, dass er eine Zwischenschicht aus Polyacrylamidgel mit einem kontrollierten Vernetzungsgrad, enthaltend spezifische Antikörper von Lipoprotein Lpa, umfasst, welche Zwischenschicht zwischen der Abtrennschicht und der Elektroimmundiffusionsschicht vorgesehen ist.